# EUROPEAN PATENT APPLICATION

(11) **EP 4 491 178 A1**
(43) Date of publication of application: **15.01.2025**
(21) Application number: 23766011.3
(22) Date of filing: 07.03.2023
(51) Int. Cl.: A61K 9/51, A61K 47/34, A61K 47/32, A61K 47/36, A61K 47/42, A61K 47/28, A61K 47/44, A61K 47/38

(54) **POLYMER-COATED NANOPARTICLES AND PREPARATION METHOD THEREFOR**

(30) Priority: 07.03.2022 CN 202210216401
(71) Applicant: Scindy Pharmaceutical (Suzhou) Co., Ltd, Suzhou, Jiangsu 215124 (CN)
(72) Inventor: CHEN, Xiaobao, Suzhou, Jiangsu 215124 (CN); LIU, Li, Suzhou, Jiangsu 215124 (CN); YUAN, Yuan, Suzhou, Jiangsu 215124 (CN); ZHANG, Wei, Suzhou, Jiangsu 215124 (CN)
(74) Representative: CH Kilger Anwaltspartnerschaft mbB
(86) International application number: PCT/CN2023/080143
(87) International publication number: WO 2023/169433

(57) **Abstract**

Polymer-coated nanoparticles and a preparation method therefor. The structural makeup of the polymer nanoparticles is: a hydrophilic aggregate inner core and a polymer molecular material coating containing a polyoxyethylene structural unit. The hydrophilic aggregate inner core comprises the following preparatory raw materials: a cationic component and an anionic component, the cationic component being a cation, a compound which can be ionized into a cation, or a composition thereof, and the anionic component being an anion, a compound which can be ionized into an anion, or a composition thereof.

## Description

The present invention claims priority to a Chinese Patent Application No. 202210216401.5, filed with the China National Intellectual Property Administration on March 7, 2022 and entitled "Polymer-coated nanoparticles and method for preparing same", the entire contents of which are incorporated herein by reference.

### Technical Field

The present invention belongs to the technical field of pharmaceutical preparations, and particularly relates to polymer-coated nanoparticles and a method for preparing polymer-coated nanoparticles.

### Background Art

Common types of nano preparation techniquesinclude liposomes, micelles, nanoparticles, microspheres, etc. At present, a drug loading technique for water-insoluble drugs is relatively mature, including structural modification, amphoteric surfactant micelle nanoparticles, liposome passive drug loading, etc. However, the nano drug loading technique for water-soluble drugs is relatively limited, and different types of techniques have strict restrictions on the solubility and ionization characteristics of drugs. For example, the permeability of liposome membranes directly affects the encapsulation and stability of drugs. So far, there is a lack of effective universal nano drug loading technique for water-soluble drugs.

Liposomes are spherical closed vesicles formed by phospholipids that encapsulate a part of the aqueous phase. A liposome usually contains two layers of phospholipid membranes, which separate an internal water space from the outside world, and its particle size can range from 20 nm to tens of microns. Among the liposome drugs currently on the market, only a few are water-soluble drugs, such as doxorubicin liposome (Doxil^{®}), vincristine liposome (Marqibo^{®}), irinotecan liposome (Onivyde^{®}), and daunorubicin and cytarabine liposome (Vyxeos^{®}), are approved for clinical indications such as anti-tumor. These liposomes are prepared by active drug loading methods such as pH gradient loading method/ammonium salt gradient method, and water-soluble drug molecules are loaded into liposomes using transmembrane gradients. These methods have strict requirements on the properties of encapsulated water-soluble drugs, requiring the drugs to have dissociable groups near physiological pH, have a suitable oil/water distribution coefficient, be weakly acidic or weakly alkaline, and form relatively stable complexes or precipitations with an internal phase buffer of liposomes (Liposome Technique, edited by Deng Yingjie, People's Medical Publishing House, 2007.1).

Polymeric micelles are spherical structures formed by amphiphilic molecules with hydrophilic and hydrophobic ends or surfactants (including ionic or non-ionic surfactants), which encapsulate hydrophobic drugs in the core. Currently, anti-cancer drug micelles such as paclitaxel and doxorubicin have entered clinical trials or are on the market. Compared with liposomes, polymeric micelles can encapsulate a large amount of hydrophobic drugs in a hydrophobic core, but are difficult to encapsulate hydrophilic drugs.

Nanoparticles also include paclitaxel-albumin nanoparticles, which are nanoparticles formed by combining biological macromolecular albumin as a carrier material with paclitaxel. Although these nanoparticles have achieved good clinical results, the nanoparticles still have problems such as strict preparation requirements and difficulties in industrialization.

Microspheres are tiny spheres or quasi-spheres formed by dissolving or dispersing drugs in polymer materials, with a particle size generally ranging from 1 µm to 250 µm. Commonly used polymer materials are water-insoluble biodegradable polymers such as polylactic acid (PLA) and poly(lactic-co-glycolic acid) (PLGA). Due to limitations of an encapsulation preparation process, it is difficult to prepare water-soluble drug nanoparticles with a particle size below 200 nm.

Taking a nano preparation of water-soluble polypeptide polymyxin as an example, the application of polymyxin liposomes has always been limited by their low encapsulation efficiency. In the related art, the encapsulation efficiency of liposomes is only less than 50%, and the polymyxin liposomes leak rapidly as a drug is diluted. The reasons why it is difficult to prepare polymyxin liposomes with high encapsulation efficiency are as follows. (1) Liposome membranes are semipermeable membranes with fluidity, and water-soluble components can easily pass through phospholipid membranes. (2) Liposome membranes have non-selective permeability to a variety of ions, and an active drug loading method driven by ion concentration is not suitable for the encapsulation of polymyxin.

Polymer nanoparticles (such as polylactic acid) are an important type of polypeptide nanocarriers that can be used to prepare local sustained-release nano-drug delivery systems for antimicrobial peptides. Due to the extremely high water solubility of polymyxin, polymyxin diffuses rapidly into an external aqueous phase during an encapsulation process of polymer nanoparticles, resulting in an extremely low encapsulation efficiency of polymyxin in polymer nanoparticles.

Currently, many nanoparticle platforms have been used for the development of water-soluble drugs, including liposomes, polymer nanoparticles, etc. However, in many cases, common problems similar to those of water-soluble drug nano preparations arise, such as low encapsulation efficiency, poor stability, or reduced drug activity.

Nano drug loading systems involved in the disclosed techniquesare mostly prepared by a thin film dispersion method, a reverse phase evaporation method or a high pressure homogenization method, and the prepared microparticles are mostly liposomes or nanoparticles, which cannot take into account the characteristics of uniform particle size distribution, good encapsulation efficiency, good stability, effective drug release, improved drug efficacy and reduced toxicity. Therefore, it is critical to develop a new nanocarrier system with good encapsulation efficiency and uniform particle size distribution to deliver drugs so as to improve the drug efficacy and drug safety.

### Summary of the Invention

In order to overcome the above technical problems, the present invention provides polymer-coated nanoparticles and a method for preparing polymer-coated nanoparticles. The polymer nanoparticles can effectively improve the drug efficacy and drug safety.

In order to achieve the above object, the present invention provides the following technical solutions.

Polymer-coated nanoparticles having a structure comprising a hydrophilic coacervate core and a polymer molecular material coating containing a polyoxyethylene structural unit, in which the hydrophilic coacervate core includes preparation raw materials of a cationic component and an anionic component, the cationic component is a cation, or a compound that is ionizable into cations or a combination thereof, and the anionic component is an anion, or a compound that is ionizable into anions or a combination thereof.

Preferably, the nanoparticles have a particle size of 10 nm to 400 nm, a PDI of less than 0.5, preferably 10 nm to 200 nm, a weakly charged or electrically neutral surface, a surface potential range of -40 mV to 40 mV, and an encapsulation efficiency greater than 60%.

Preferably, the encapsulation efficiency of the nanoparticles is greater than 60%, preferably greater than 70%.

Preferably, the cationic compound or the combination thereof is any one or more of ionizable or net positively charged hydrophilic small molecule compounds, polypeptides, polymers and phospholipids.

Preferably, the polypeptide is an ionizable or net positively charged hydrophilic polypeptide with a molecular weight of less than 10,000.

Preferably, the polypeptide include any one or more of an ionizable hydrophilic polypeptide with a cyclic structure containing a free amine group and derivatives thereof, an ionizable hydrophilic polypeptide with a molecular weight less than 10,000 and having a free primary amine, secondary amine, or tertiary amine structure and derivatives thereof, polylysine and derivatives thereof, polyarginine and derivatives thereof, polyhistidine and derivatives thereof, and arginine-rich polypeptide.

Preferably, the polypeptide is any one or more of polymyxin and derivatives thereof, vancomycin, tigecycline, capreomycin, teicoplanin, daptomycin, caspofungin, micafungin, glucagon, exenatide, pramlintide, dulaglutide, liraglutide, semaglutide, albiglutide, insulin, romidepsin, actinomycin, gonadorelin, bleomycin, leuprorelin, goserelin, histrelin, triptorelin, buserelin, eptifibatide, icatibant, nesiritide, octreotide, linaclotide, somatostatin, terlipressin, atosiban, carbetocin, oxytocin, lysine vasopressin, vasopressin, desmopressin, cobicistat, thymopentin, glutathione, enfuvirtide, thymalfasin, tesamorelin, glatiramer acetate, bivalirudin, ziconotide, pasireotide, etelcalcetide, teriparatide, and abaloparatide.

Preferably, the polymyxin and derivatives thereof include any one or more of polymyxin B sulfate, polymyxin E, polymyxin E1, polymyxin E2, polymyxin E sodium methanesulfonate, polymyxin E1 sodium methanesulfonate, and polymyxin E2 sodium methanesulfonate.

Preferably, the ionizable or net positively charged hydrophilic small molecule compound is an ionizable or net positively charged hydrophilic compound containing an amine structure.

Preferably, the ionizable or net positively charged hydrophilic small molecule compounds include any one or more of a metal complex and derivatives thereof, an ionizable hydrophilic anesthetic/analgesic drug containing an amine structure, an ionizable hydrophilic psychotropic drug containing an amine structure, and an ionizable hydrophilic anti-cancer and anti-tumor drug containing an amine structure and derivatives thereof.

Preferably, the ionizable or net positively charged hydrophilic small molecule compound is any one or more of lenalidomide, pomalidomide, dabrafenib, carfilzomib, acalabrutinib, palbociclib, trametinib, bortezomib, ruxolitinib, imatinib, sunitinib, axitinib, ribociclib, capecitabine, osimertinib, minoxidil, primidone, procainamide hydrochloride, flecainide, phenytoin sodium, guanethidine monosulfate, isoprenaline sulphate, clindamycin hydrochloride, clonidine hydrochloride, theophylline, aminophylline, quinidine sulfate, metformin, alogliptin, saxagliptin, linagliptin, vildagliptin, teneligliptin, hydrochlorothiazide, hydralazine, labetalol, metoprolol, clonidine, nicardipine, esmolol, torasemide, valsartan, diltiazem, phentolamine, salbutamol, salmeterol, lacosamide, tiotropium Bromide, olanzapine, levetiracetam, methylphenidate, bupropion hydrochloride, brivaracetam, lamotrigine, cariprazine, chlorpromazine, haloperidol, risperidone, quetiapine, citalopram, paliperidone palmitate, mirtazapine, chlormezazone, pregabalin, parecoxib, bupivacaine, pentazocine, butorphanol, naloxone, codeine, pethidine, fentanyl, dihydroetorphine, tramadol, morphine, ketorolac tromethamine, remdesivir, emtricitabine, tenofovir disoproxil, lamivudine, amikacin sulfate, imipenem, darunavir, clavulanic acid, fingolimod, lisdexamfetamine, benserazide, droxidopa, meta-iodobenzylguanidine, iopromide, iohexol, carboplatin, cisplatin, oxaliplatin, [SP-4-2-[(1R-trans)]-(1,2-Cyclohexanediamine-N,N') dinitratoplatinum, platinum cyclohexanediamine dihydrate, ormaplatin and other platinum complexes and derivatives thereof, ruthenium (II) complexes and derivatives thereof, vanadium complexes and derivatives thereof, myristyltrimethylammonium bromide, imidazolidinyl urea, meglumine derivatives, and triethanolamine derivatives.

Preferably, the polymer is any one or more of a hydrophilic and ionizable polymer containing an amine group, guanidine group or imine group in a side chain or a main chain, and a cationic polysaccharide.

Preferably, the polymer is any one or more of polyethyleneimine (PEI), polyamidoamine (PAMAM), poly(beta-amino ester), polyamine coester, chitosan and derivatives thereof, and povidone.

Preferably, the phospholipid is any one or more of cationic lipids and derivatives thereof whose hydrophilic head group contains a quaternary ammonium group, a pyridyl group, a guanidine group, an imidazole group, ionizable lipids and derivatives thereof whose hydrophilic head group contain an amine structure, polyethyleneimine-phospholipid and derivatives thereof, and cholesteryl 3β-N-(dimethylaminoethyl)carbamate (DC-CHOL) and derivatives thereof.

Preferably, the anionic compound or the combination thereof is any one or more of an ionizable or anionic hydrophilic small molecule compound, an ionizable or anionic hydrophilic monosaccharide or oligosaccharide acid and derivatives thereof, an ionizable or anionic hydrophilic polysaccharide and derivatives thereof, an anionic polyamino acid and derivatives thereof, and a nucleic acid.

Preferably, the monosaccharide or oligosaccharide acid and the derivatives thereof include any one or more of sulfated glucose, sulfated fructose, phosphorylated glucose, phosphorylated fructose, sulfated sucrose, sulfated lactose, sulfated trehalose, phosphorylated sucrose, phosphorylated lactose and phosphorylated trehalose.

Preferably, the polysaccharide and the derivatives thereof are any one or more of glycosaminoglycan, cellulose polysaccharide, polyuronic acid polysaccharide, and galactomannan polysaccharide and derivatives thereof.

Preferably, the polysaccharide and the derivatives thereof are any one or more of sodium hyaluronate, cross-linked sodium hyaluronate, chondroitin sulfate, dermatan sulfate, keratan sulfate, heparin sodium, heparan sulfate, dalteparin sodium, enoxaparin sodium, and fondaparinux sodium.

Preferably, the polysaccharide and the derivatives thereof are any one or more of cellulose acetate, sodium carboxymethyl cellulose, hydroxyethyl cellulose, hydroxyethyl methyl cellulose, hydroxypropyl cellulose and hydroxypropyl methyl cellulose.

Preferably, the polysaccharide and the derivatives thereof are any one or more of polyglucuronic acid, polyuronide, sodium polymannosuronate, sodium polyguluronate, alginic acid, sodium alginate, propylene glycol alginate, xanthan gum, tragacanth gum, arabic gum, and carrageenan. Preferably, the nucleic acid is ribonucleic acid or deoxyribonucleic acid.

Preferably, the nucleic acid is siRNA, mRNA, miRNA, antisense oligonucleotide, DNA, circular RNA, tRNA.

Preferably, the polyoxyethylene structural unit contained is -(CH₂CH₂O)n, where n = 6 to 460.

Preferably, the polymer molecular material containing a polyoxyethylene structural unit is any one or more of PEG-phospholipid, PEG-cholesterol, PEG-polymer, polysorbate, polyoxyethylene castor oil and derivatives thereof, and polyoxyethylene stearate.

Preferably, the polymer molecular material containing a polyoxyethylene structural unit is any one or more of polyethylene glycol, polyethylene glycol-polylactic acid, polyethylene glycol-polylactic acid-glycolic acid polymer, polyethylene glycol-polyglutamic acid, polyglutamic acid-polyethylene glycol-carboxylic acid, polyethylene glycol-1,2-distearoyl-sn-glycero-3-phosphoethanolamine, polyethylene glycol-dimyristoyl-sn-glycerol, polyethylene glycol-distearoyl glycerol, polyethylene glycol-dimyristoyl phosphatidylethanolamine, polyethylene glycol-dipalmitin, polyethylene glycol-dipalmitoyl phosphatidylethanolamine, polyethylene glycol-dilauroyl phosphatidylethanolamine, polyethylene glycol-dipalmitoyl phosphatidylserine, polyethylene glycol-dioleoyl phosphatidylethanolamine, polyethylene glycol-cholesterol, and cholesterol-polyethylene glycol-VA.

Preferably, the cationic component includes any one or more of polymyxin and derivatives thereof, a platinum complex and derivatives thereof, a ruthenium (II) complex and derivatives thereof, a vanadium complex and derivatives thereof, DC-cholesterol and derivatives thereof, chitosan hydrochloride and derivatives thereof, polyethyleneimine hydrochloride and derivatives thereof, poly-L-arginine hydrochloride and derivatives thereof, 1,2-dioleoyl-3-trimethylammonium propane (DOTAP), N,N,N-Trimethyl-2,3-bis(octadec-9-en-1-yloxy)propan-1-aminium chloride (DOTMA), thymalfasin, octreotide acetate, liraglutide, pasireotide, risperidone hydrochloride, clonidine hydrochloride, methylphenidate, brivaracetam, levetiracetam, pregabalin, bortezomib, imatinib, palbociclib, daptomycin, vancomycin, tigecycline, imipenem, clindamycin hydrochloride, and amikacin sulfate.

Preferably, the anionic component includes any one or more of polyglutamic acid polypeptide and derivatives thereof, polyaspartic acid polypeptide and derivatives thereof, hyaluronate, sucrose octasulfate, sodium alginate, xanthan gum, CMC-Na, siRNA, mRNA, sulfadiazine sodium, and sodium carboxymethyl cellulose.

Preferably, the polymer molecular material containing a polyoxyethylene structural unit includes any one or more of 1,2-distearoyl-rac-glycero-3-PE-N-polyethyleneglycol, PEG-PLA, (polyethylene glycol-polylactic acid block copolymer), 1,2-dimyristoyl-rac-glycero-3-methoxypolyethylene glycol, polyethylene glycol-polylactic acid-glycolic acid polymer, polyglutamic acid-polyethylene glycol-carboxylic acid, polysorbate, polyoxyethylene castor oil, polyoxyethylene stearate, polyethylene glycol-polyglutamic acid, polyethylene glycol-dipalmitin, polyethylene glycol-dipalmitoyl phosphatidylethanolamine, polyethylene glycol-dimyristoyl phosphatidylethanolamine, polyethylene glycol-dilauroyl phosphatidylethanolamine, polyethylene glycol-dipalmitoyl phosphatidylserine, polyethylene glycol-cholesterol, and cholesterol-polyethylene glycol-VA.

Preferably, in the nanoparticles, a charge ratio of the cationic component to the anionic component is 1 to 8:1 to 10.

Preferably, a mass percentage of the hydrophilic coacervate core in the nanoparticles ranges from 1% to 90%, preferably from 5% to 80%.

Another object of the present invention is to provide a method for preparing the nanoparticles, the method including:
(1) dissolving and mixing the cationic component and the anionic component to obtain a coacervate solution;
(2) dissolving the polymer molecular material containing a polyoxyethylene structural unit to obtain a coating solution; and
(3) mixing the coacervate solution and the coating solution to obtain a polymer-coated nanoparticle solution.

Preferably, the polymer-coated nanoparticle solution may be further filtered and/or purified.

Preferably, during the preparation of the nanoparticles, the coacervate solution is a homogeneous solution.

Preferably, in the method for preparing the nanoparticles, the mixing adopts a microchannel mixing method, and the microchannel mixing method adopts an injection ttechnique, a microfluidic technique or a jet flow technique.

Preferably, a mixing volume ratio of the coacervate solution to the coating solution is 1 to 10: 1 to 20, preferably 1 to 8:1 to 10.

Preferably, a mixing speed of the microchannel mixing is 0.5 ml/min to 400 ml/min.

Preferably, in the method for preparing the nanoparticles, the injection technique is to inject the coacervate solution into the coating solution or the coating solution into the coacervate solution.

Preferably, the nanoparticles are freeze-dried for storage.

Another object of the present invention is to provide a preparation of the nanoparticles. A structure of the nanoparticles is characterized by a hydrophilic coacervate core containing an active ingredient and a polymer molecule coating containing a polyoxyethylene structural unit. The nanoparticles have an encapsulation efficiency greater than 60%, an average particle size of 10 nm to 400 nm, a weakly charged or electrically neutral surface, and a surface potential range of -40 mV to 40 mV.

Preferably, the preparation of the nanoparticles further contains an excipient commonly used in pharmaceutics. The excipient includes any one or more of potassium dihydrogen phosphate, disodium hydrogen phosphate, sodium dihydrogen phosphate, dipotassium hydrogen phosphate, sodium chloride, potassium chloride, sodium acetate, acetic acid, sucrose, mannitol, lactose, glucose, trehalose, polyethylene glycol, glycerol, phosphate, acetate, amino acid, water for injection, a 0.5% to 0.9% sodium chloride solution (preferably 0.9% sodium chloride solution), a 1% to 5% glucose solution (preferably 5% glucose solution), and a PBS buffer.

Administration routes of the preparation of nanoparticles provided in the present invention includes subcutaneous, intradermal, intramuscular, joint, thoracic, spinal cavity, intralesional, intracranial, intravenous injection and any suitable injection method, and pulmonary administration.

Compared with the related art, the present invention has the following technical advantages.
(1) The present invention provides a preparation of nanoparticles with good performance. The selection of materials in the preparation of nanoparticles has a certain influence on the encapsulation efficiency, particle size, morphology, in vivo stability, drug release rate, drug efficacy and toxicity of the nanoparticles.
(2) Commonly used nano drug delivery systems (liposomes, micelles, inorganic nanoparticles, polymer nanoparticles, etc.) are usually prepared by a thin film dispersion method, a reverse phase evaporation method or a high pressure homogenization method. When the preparation involves a mixing step, the macroscopic fluid interaction principle is often used for the preparation. A preparation process is relatively cumbersome and there are structural differences between batches. The particle size distribution of the obtained nanoparticles is uneven, and the dispersion and repeatability are poor. The present invention utilizes a microchannel mixing technique to precisely control a flow rate of a fluid, so that liquid components introduced at different times can be fully mixed and are highly uniform and ordered. The prepared nanoparticles show obvious advantages in terms of particle structure uniformity, batch-to-batch repeatability, and drug loading rate.
(3) Compared with the related art (nano preparations such as liposomes, micelles, and microspheres), the nanoparticles and the method for preparing the nanoparticles of the present invention form a hydrophilic coacervate core with a hydrophilic active ingredient and a hydrophilic polymer material. The nanoparticles are coated with a hydrophilic amphiphilic polymer material containing a polyoxyethylene structure, thereby providing spherical nanoparticles containing a hydrophilic coacervate core and a polymer molecule coating containing a polyoxyethylene structural unit. The hydrophilic coacervate core relies on intermolecular forces including charge interaction (Coulomb's law) and spatial effect to form a hydrophilic coacervate. The hydrophilic coacervate is a homogeneous solution at the macroscopic level and is in a liquid-liquid phase separation state at the microscopic level.
(4) The nanoparticles provided by the present invention maintain the efficacy of free hydrophilic drugs while significantly improving the safety.
(5) The nanoparticle preparation process provided by the present invention adopts a precise mixing process of an aqueous solution, and does not need to adopt traditional nano preparation processes such as an organic solvent thin film dispersion method and a high pressure homogenization method. The process is simple, has good reproducibility, and is easy to realize commercial production.

### Brief Description of Drawings

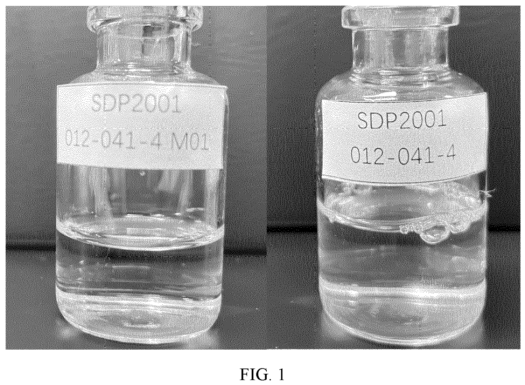

FIG. 1 is a diagram of a hydrophilic coacervate core solution (left) and a polymer-coated nanoparticle solution (right) in Example 1.

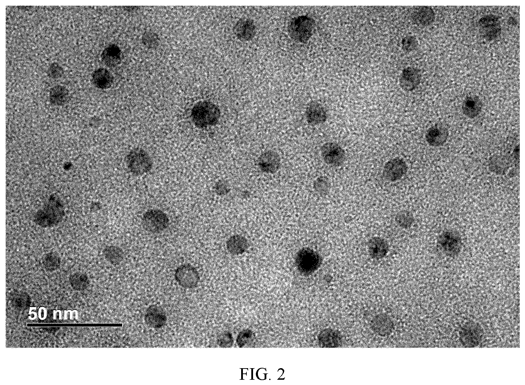

FIG. 2 is a transmission electron micrograph of polymer-coated nanoparticles of Example 4.

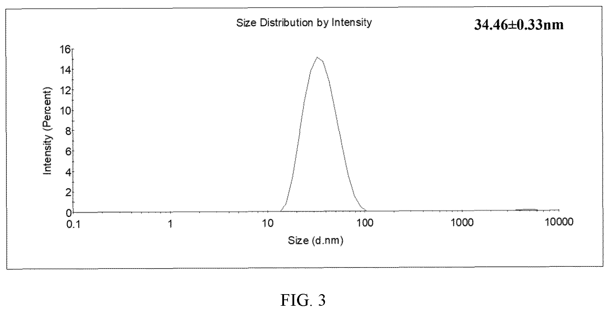

FIG. 3 is a diagram showing an average particle size of polymer-coated nanoparticles of Example 1.

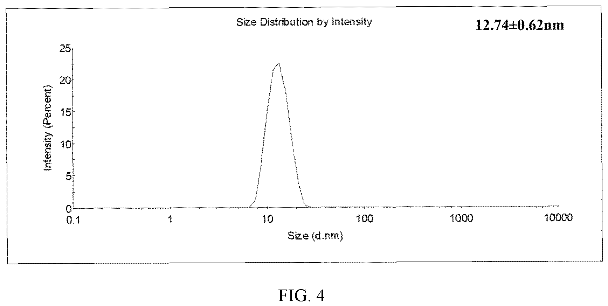

FIG. 4 is a diagram showing an average particle size of polymer-coated nanoparticles of Example 4.

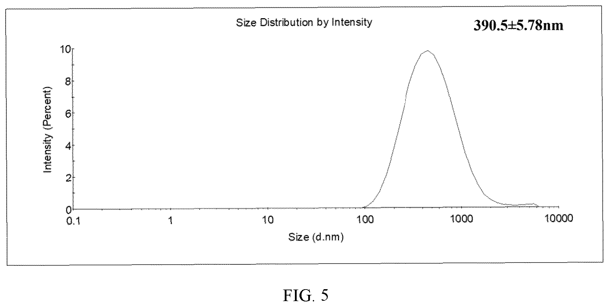

FIG. 5 is a diagram showing an average particle size of polymer-coated nanoparticles of Example 8.

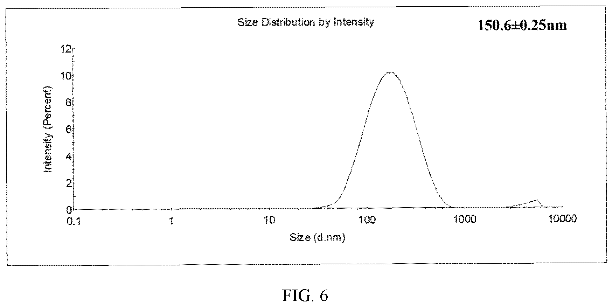

FIG. 6 is a diagram showing an average particle size of polymer-coated nanoparticles of Example 12.

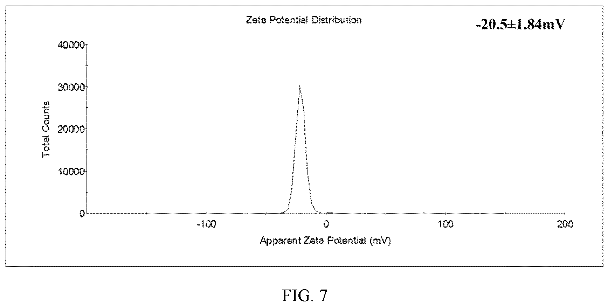

FIG. 7 shows the surface potential of polymer-coated nanoparticles of Example 5.

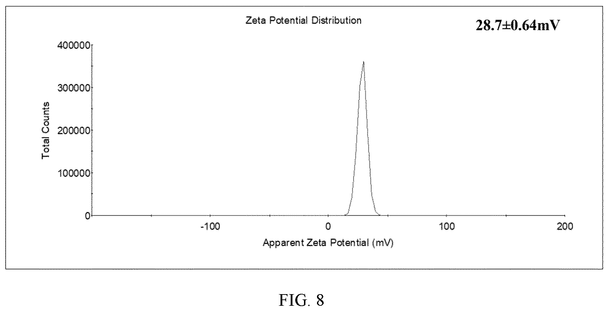

FIG. 8 shows the surface potential of polymer-coated nanoparticles of Example 8.

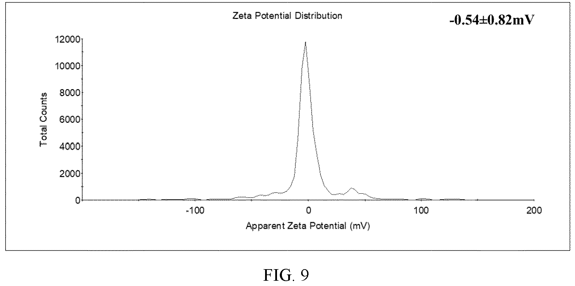

FIG. 9 shows the surface potential of polymer-coated nanoparticles of Example 10.
FIG. 10 shows a release curve of active ingredients in polymer-coated nanoparticles.

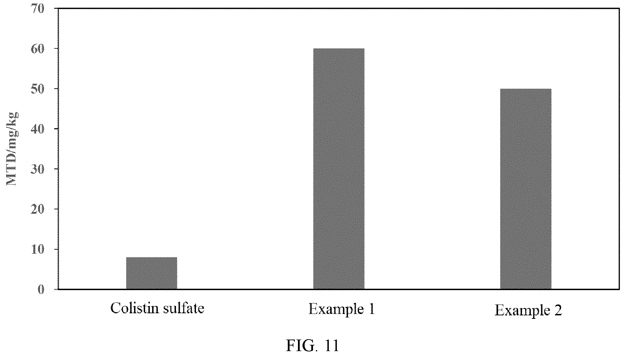

FIG. 11 is an MTD diagram of a safety test of polymer-coated nanoparticles of Examples 1 and 2.

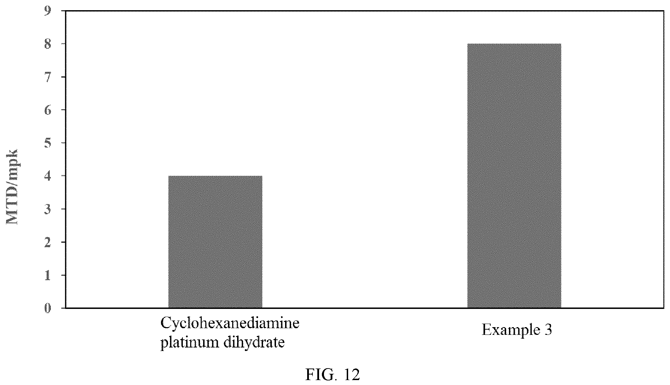

FIG. 12 is an MTD diagram of a safety test of polymer-coated nanoparticles of Example 3.

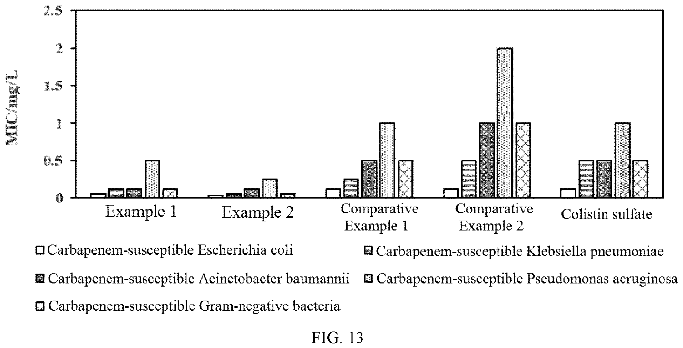

FIG. 13 is an MIC diagram of an antibacterial test of polymer-coated nanoparticles.

### Detailed Description

The present invention will be further described in detail below in conjunction with specific examples. The following examples are not intended to limit the present invention, but are only intended to illustrate the present invention. Unless otherwise specified, the experimental methods used in the following examples, as well as the experimental methods without specific conditions indicated in the examples, are generally carried out under conditions in the related art. The materials, reagents, etc. used in the following examples are all commercially available unless otherwise specified.

### Example 1

**Preparation method:** In 10 ml of water, 20 mg of colistin sulfate and 32 mg of polyglutamic acid were dissolved to obtain a solution 1. In 30 ml of water, 600 mg of mPEG5000-DSPE (1,2-distearoyl-sn-glycero-3-phosphoethanolamine-N-[methoxy(polyethylene glycol)-5000]) was dissolved to obtain a solution 2. The solution 1 was injected into the solution 2 at a rate of 12 ml/min for mixing, and the mixed solution was sterilized and filtered to obtain colistin polymer-coated nanoparticles.

**Detection method:** Malvern ZetaSizer Nano ZS90 was used to measure an average particle size and a polydispersity index (PDI) of the polymer-coated nanoparticles of the example by dynamic light scattering. The measuring angle was 90°, the dispersant refractive index was 1.330, and the test temperature was 25°C.

Malvern ZetaSizer Nano ZS90 was used to measure the Zeta potential of the polymer-coated nanoparticles of the example based on an electrophoretic light scattering technique(ELS). The dispersant refractive index was 1.330, and the test temperature was 25°C.

Into an ultrafiltration centrifuge tube (Amicon Ultra-4 Centrifugal Filters, molecular weight cut-off: 30 kD), 2.5 ml of the polymer-coated nanoparticle solution in the example was loaded, and the solution was centrifuged at 5000 rpm/min. The amount of free drug in the centrifuge tube and the amount of encapsulated drug retained on a filter membrane were measured using an HLPC method. The measurement was performed with 4.46 g/L sodium sulfate solution (pH 2.4)-acetonitrile (78: 22) as a mobile phase, at a detection wavelength of 215 nm, a flow rate of 1.0 ml/min, and an injection volume of 20 µl. The measured amount of retained drug was 1.2484 mg and the measured amount of free drug was 0.0044630 mg. (The encapsulation efficiency calculation formula is: encapsulation efficiency (EE%) = amount of retained drug/(amount of retained drug + amount of free drug)×100%), and the results are shown in Table 3 below.

### Example 2

**Preparation method:** In 20 ml of water, 15 mg of polymyxin B sulfate and 54 mg of sodium carboxymethyl cellulose were dissolved to obtain a solution 1. In 10 ml of water, 186 mg of polyoxyethylene stearate was dissolved to obtain a solution 2. The solution 1 and the solution 2 were mixed at a rate of 20 ml/min using a microfluidic device. The mixed solution was sterilized and filtered to obtain polymyxin B polymer-coated nanoparticles.

**Detection method:** Same as Example 1. The measured amount of retained drug was 1.2074 mg, and the measured amount of free drug was 0.032871 mg. The results are shown in Table 3 below.

### Example 3

**Preparation method:** In 10 ml of water, 20 mg of platinum cyclohexanediamine dihydrate and 72 mg of sodium hyaluronate were dissolved to obtain a solution 1. In 30 ml of water, 208 mg of PEG-cholesterol was dissolved to obtain a solution 2. The solution 1 and the solution 2 were mixed at a rate of 9 ml/min using a microfluidic device. The mixed solution was sterilized and filtered to obtain platinum cyclohexanediamine dihydrate polymer-coated nanoparticles.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 3 kD), 1.5 ml of the polymer-coated nanoparticle solution in the example was loaded, and the solution was centrifuged at 5000 rpm/min to collect the free drug in the centrifuge tube. The total drug concentration and the free drug concentration were measured by ICP-MS. The measured total drug concentration was 0.51247 mg/ml, and the measured free drug concentration was 0.065258 mg/ml. (The encapsulation efficiency calculation formula is: encapsulation efficiency (EE%) = (total drug concentration - free drug concentration)/total drug concentration × 100%), and the results are shown in Table 3 below.

### Example 4

**Preparation method:** In 10 ml of water, 14 mg of amikacin sulfate and 17 mg of sucrose octasulfate sodium salt were dissolved to obtain a solution 1. In 30 ml of water, 129 mg of polysorbate 20 was dissolved to obtain a solution 2. The solution 1 and the solution 2 were mixed at a rate of 6 ml/min using a microfluidic device. The mixed solution was sterilized and filtered to obtain amikacin polymer-coated nanoparticles.

**Stability investigation:** Physiological conditions (pH 7.4, 10 mM phosphate + 150 mM NaCl) were simulated, and freeze-dried powder of the polymer-coated nanoparticles of the example was reconstituted with a solution under the simulated physiological conditions and placed at 37°C for 3 days to investigate the in vitro stability of the polymer-coated nanoparticles.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 3 kD), 1.5 ml of the polymer-coated nanoparticle solution in the example was loaded, and the solution was centrifuged at 5000 rpm/min to collect the free drug in the centrifuge tube. The total drug concentration and the concentration of the free drug in the centrifuge tube were measured using an HLPC method. A mobile phase consisted of 80% aqueous solution and 20% acetonitrile. The aqueous phase was prepared by dissolving potassium dihydrogen phosphate (1.36 g) and sodium 1-heptanesulfonate (1 g) in 1000 ml of ultrapure water, and the pH was adjusted to 3.0 with phosphoric acid. The measurement was performed at a detection wavelength of 200 nm, a flow rate of 1.0 ml/min, and an injection volume of 20 µl. The measured total drug concentration was 0.35452 mg/ml (0 days) and 0.36405 mg/ml (3 days), and the measured free drug concentration was 0.0020755 mg/ml (0 days) and 0.0052642 mg/ml (3 days). (The encapsulation efficiency calculation formula is: encapsulation efficiency (EE%) = (total drug concentration - free drug concentration)/total drug concentration × 100%), and the results are shown in Table 3 below.

### Example 5

**Preparation method:** In 10 ml of water, 38 mg of octreotide acetate and 18 mg of sodium alginate were dissolved to obtain a solution 1. In 10 ml of water, 214 mg of ethoxylated hydrogenated castor oil was dissolved to obtain a solution 2. The solution 1 and the solution 2 were mixed at a rate of 2 ml/min using a microfluidic device. The mixed solution was sterilized and filtered to obtain octreotide polymer-coated nanoparticles.

Stability investigation: Same as Example 4.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 3 kD), 1.5 ml of the polymer-coated nanoparticle solution in the example was loaded, and the solution was centrifuged at 5000 rpm/min to collect the free drug in the centrifuge tube. The total drug concentration and the concentration of the free drug in the centrifuge tube were measured using an HLPC method. Tetramethylammonium hydroxide solution (880 ml of water was added to 20 ml of 10% tetramethylammonium hydroxide solution, and the pH value was adjusted to 5.4 with 10% phosphoric acid solution)-acetonitrile (900: 100) was used as a mobile phase A, and tetramethylammonium hydroxide solution (380 ml of water was added to 20 ml of 10% tetramethylammonium hydroxide solution, and the pH value was adjusted to 5.4 with 10% phosphoric acid)-acetonitrile (400: 600) was used as a mobile phase B. The flow rate was 1.0 ml/min, and gradient elution was performed according to Table 1 below. The measurement was performed at a detection wavelength of 210 nm, a flow rate of 1.0 ml/min, and an injection volume of 20 µl. The measured total drug concentration was 1.9182 mg/ml (0 days) and 1.8937 mg/ml (3 days), and the measured free drug concentration was 0.040467 mg/ml (0 days) and 0.065353 mg/ml (3 days). (The encapsulation efficiency calculation formula is: encapsulation efficiency (EE%) = (total drug concentration - free drug concentration)/total drug concentration × 100%), and the results are shown in Table 3 below.

**TABLE 1 Gradient elution**

| Time (minutes) | Mobile phase A (%) | Mobile phase B (%) |
|---|---|---|
| 0 | 73 | 27 |
| 30 | 55 | 45 |
| 31 | 73 | 27 |
| 37 | 73 | 27 |

### Example 6

**Preparation method:** In 10 ml of water, 24 mg of clindamycin hydrochloride and 72 mg of xanthan gum were dissolved to obtain a solution 1. In 20 ml of water, 156 mg of PEG-PLA was dissolved to obtain a solution 2. The solution 1 and the solution 2 were mixed at a rate of 16 ml/min using a jet flow device. The mixed solution was sterilized and filtered to obtain clindamycin polymer-coated nanoparticles.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 30 kD), 1.5 ml of the polymer-coated nanoparticle solution in the example was loaded, and the solution was centrifuged at 5000 rpm/min to collect the free drug in the centrifuge tube. The total drug concentration and the concentration of the free drug in the centrifuge tube were measured using an HLPC method. The measurement was performed with 6.8 g/L potassium dihydrogen phosphate (pH 7.5)-acetonitrile (55: 45) as a mobile phase, at a flow rate of 1.0 ml/min, a detection wavelength of 210 nm, a flow rate of 1.0 ml/min, and an injection volume of 20 µl. The measured total drug concentration was 0.81561 mg/ml, and the measured free drug concentration was 0.012101 mg/ml. (The encapsulation efficiency calculation formula is encapsulation efficiency (EE%) = (total drug concentration - free drug concentration)/total drug concentration × 100%), and the results are shown in Table 3 below.

### Example 7

**Preparation method:** In 30 ml of water, 57 mg of thymalfasin and 72 mg of CMC-Na were dissolved to obtain a solution 1. In 10 ml of water, 292 mg of mPEG2000-DSPE (1,2-distearoyl-rac-glycero-3-PE-N-polyethyleneglycol-2000) was dissolved to obtain a solution 2. The solution 1 and the solution 2 were mixed at a rate of 12 ml/min using a microfluidic device. The mixed solution was sterilized and filtered to obtain thymalfasin polymer-coated nanoparticles.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 30 kD), 1.5 ml of the polymer-coated nanoparticle solution in the example was loaded, and the solution was centrifuged at 5000 rpm/min to collect the free drug in the centrifuge tube. The total drug concentration and free drug concentration were measured using an HLPC method. Ammonium phosphate buffer (26.4 g of ammonium sulfate and 25 ml of phosphoric acid were dissolved in water and diluted to 2000 ml)-acetonitrile (90: 10) was used as a mobile phase A, and ammonium sulfate buffer-acetonitrile (50: 50) was used as a mobile phase B. The flow rate was 1.0 ml/min, and gradient elution was performed according to Table 2 below. The measurement was performed at a detection wavelength of 210 nm, a column temperature of 50°C, and an injection volume of 20 µl. The measured total drug concentration was 1.4209 mg/ml, and the measured free drug concentration was 0.0033155 mg/ml. (The encapsulation efficiency calculation formula is: encapsulation efficiency (EE%) = (total drug concentration - free drug concentration)/total drug concentration × 100%), and the results are shown in Table 3 below.

**TABLE 2 Gradient elution**

| Time (minutes) | Mobile phase A (%) | Mobile phase B (%) |
|---|---|---|
| 0 | 88 | 12 |
| 45 | 82 | 18 |
| 50 | 50 | 50 |
| 51 | 88 | 12 |
| 60 | 88 | 12 |

### Example 8

**Preparation method:** In 10 ml of a 0.3 mg/ml siRNA solution, 12 mg of DC-cholesterol was dissolved to obtain a solution 1. In 10 ml of water, 32 mg of DMG-PEG2000 (1,2-dimyristoyl-rac-glycero-3-methoxypolyethylene glycol-2000) was dissolved to obtain a solution 2. The solution 1 and the solution 2 were mixed at a rate of 12 ml/min using a microfluidic device. The mixed solution was sterilized and filtered to obtain siRNA polymer-coated nanoparticles.

Stability investigation: Same as Example 4.

**Detection method:** Particle size, PDI, and potential detection methods are the same as in Example 1, and an encapsulation efficiency detection method is as follows.

An appropriate amount of the polymer-coated nanoparticle solution in the example was centrifuged at 10000g centrifugal force for 30 minutes to collect the supernatant (free drug). The demulsified polymer-coated nanoparticle solution and the supernatant were mixed with a Qubit detection reagent, respectively, and then the total drug concentration and the free drug concentration were measured using an Invitrogen Qubit 4 fluorescence quantitative instrument. The measured total drug concentration was 0.15385 mg/ml (0 days) and 0.14840 mg/ml (3 days), and the measured free drug concentration was 0.037231 mg/ml (0 days) and 0.030001 mg/ml (3 days). (The encapsulation efficiency calculation formula is: encapsulation efficiency (EE%) = (total drug concentration - free drug concentration)/total drug concentration × 100%), and the results are shown in Table 3 below.

### Example 9

**Preparation method:** In 30 ml of a 0.2 mg/ml siRNA solution, 12 mg of chitosan hydrochloride (CAS No. 70694-72-3) was dissolved to obtain a solution 1. In 10 ml of water, 52 mg of PEG-cholesterol was dissolved to obtain a solution 2. The solution 1 and the solution 2 were mixed at a rate of 6 ml/min using a microfluidic device. The mixed solution was sterilized and filtered to obtain siRNA polymer-coated nanoparticles.

**Detection method:** Same as Example 8. The measured total drug concentration was 0.15644 mg/ml, and the measured free drug concentration was 0.037077 mg/ml. The results are shown in Table 3 below.

### Example 10

**Preparation method:** In 20 ml of water, 17 mg of polyethyleneimine hydrochloride (CAS No. 49553-93-7) and 14 mg of sulfadiazine sodium were dissolved to obtain a solution 1. In 10 ml of water, 96 mg of polysorbate 20 was dissolved to obtain a solution 2. The solution 1 and the solution 2 were mixed at a rate of 24 ml/min using a microfluidic device. The mixed solution was sterilized and filtered to obtain sulfadiazine polymer-coated nanoparticles.

Stability investigation: Same as Example 4.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 3 kD), 1.5 ml of the polymer-coated nanoparticle solution in the example was loaded, and the solution was centrifuged at 5000 rpm/min to collect the free drug in the centrifuge tube. The total drug concentration and free drug concentration were measured using an HLPC method. The measurement was performed with 0.1% phosphoric acid-acetonitrile (85: 15) as a mobile phase, at a detection wavelength of 270 nm, a column temperature of 40°C, and an injection volume of 20 µl. The measured total drug concentration was 0.48057 mg/ml (0 days) and 0.48896 mg/ml (3 days), and the measured free drug concentration was 0.017063 mg/ml (0 days) and 0.018606 mg/ml (3 days). (The encapsulation efficiency calculation formula is: encapsulation efficiency (EE%) = (total drug concentration - free drug concentration)/total drug concentration × 100%), and the results are shown in Table 3 below.

### Example 11

**Preparation method:** In 30 ml of water, 32 mg of risperidone hydrochloride and 52 mg of sodium carboxymethyl cellulose were dissolved to obtain a solution 1. In 10 ml of water, 291 mg of mPEG2000-DSPE (1,2-distearoyl-rac-glycero-3-PE-N-polyethyleneglycol-2000) was dissolved to obtain a solution 2. The solution 1 and the solution 2 were mixed at a rate of 16 ml/min using a microfluidic device. The mixed solution was sterilized and filtered to obtain risperidone polymer-coated nanoparticles.

**Detection method:** Particle size, PDI, and potential detection methods are the same as in Example 1, and an encapsulation efficiency detection method is as follows.

Into an ultrafiltration centrifuge tube (Amicon Ultra-4 Centrifugal Filters, molecular weight cut-off: 30 kD), 1.5 ml of the polymer-coated nanoparticle solution in the example was loaded, and the solution was centrifuged at 5000 rpm/min to collect the free drug in the centrifuge tube. The total drug concentration and free drug concentration were measured using an HLPC method. The measurement was performed with methanol-0.05 mol/L ammonium acetate solution (the pH was adjusted to 6.8 with an ammonia test solution) (60: 40) as a mobile phase, at a detection wavelength of 275 nm, a column temperature of 40°C, and an injection volume of 20 µl. The measured total drug concentration was 0.81783 mg/ml, and the measured free drug concentration was 0.0088522 mg/ml. (The encapsulation efficiency calculation formula is: encapsulation efficiency (EE%) = (total drug concentration - free drug concentration)/total drug concentration × 100%), and the results are shown in Table 3 below.

### Example 12

**Preparation method:** In 10 ml of a 0.2 mg/ml mRNA solution, 2 mg of poly-L-arginine hydrochloride (CAS No. 26982-20-7) was dissolved to obtain a solution 1. In 10 ml of water, 17 mg of PEG-PLA was dissolved to obtain a solution 2. The solution 1 and the solution 2 were mixed at a rate of 6 ml/min using a microfluidic device. The mixed solution was sterilized and filtered to obtain mRNA polymer-coated nanoparticles.

Stability investigation: Same as Example 4.

**Detection method:** Same as Example 8. The measured total drug concentration was 0.10297 mg/ml (0 days) and 0.10196 mg/ml (3 days), and the measured free drug concentration was 0.014696 mg/ml (0 days) and 0.011788 mg/ml (3 days). The results are shown in Table 3 below.

### Example 13

**Preparation method:** In 5 ml of a 50% ethanol aqueous solution, 32 mg of DOTAP (1,2-dioleoyl-3-trimethylammonium-propane) was dissolved, and the obtained solution was mixed with 30 ml of a 0.1 mg/ml mRNA aqueous solution to obtain a solution 1. In 5 ml of water, 48 mg of mPEG2000-DSPE was dissolved to obtain a solution 2. The solution 1 and the solution 2 were mixed at a rate of 4 ml/min using a microfluidic device, and the mixed solution was diluted to 200 times the volume with a pH 7.4 PBS buffer and dialyzed twice using a 10 kD regenerated cellulose dialysis membrane to remove ethanol and perform buffer exchange. The first dialysis was performed at room temperature for 3 h, and the last dialysis was performed at 4°C overnight. The mixed solution was sterilized and filtered to obtain mRNA polymer-coated nanoparticles.

**Detection method:** Same as Example 8. The measured total drug concentration was 0.077670 mg/ml, and the measured free drug concentration was 0.0083707 mg/ml. The results are shown in Table 3 below.

### Comparative Example 1

**Preparation method:** In 40 ml of water, 20 mg of colistin sulfate was dissolved, followed by adding and dissolving 600 mg of mPEG5000-DSPE, and then continuing to add 32 mg of polyglutamic acid. The mixed solution was dispersed using a high-pressure homogenizer, and then sterilized and filtered to obtain colistin polymer-coated nanoparticles.

**Detection method:** Same as Example 1. The measured amount of retained drug was 0.52187 mg, and the measured amount of free drug was 0.77657 mg. The results are shown in Table 3 below.

### Comparative Example 2

**Preparation method:** In 20 ml of water, 20 mg of polymyxin B sulfate and 72 mg of sodium hyaluronate were dissolved, and the mixed solution was sterilized and filtered to obtain polymyxin B nanoparticles.

**Detection method:** Same as Example 1. The measured amount of retained drug was 0.61199 mg, and the measured amount of free drug was 1.8497 mg. The results are shown in Table 3 below.

### Comparative Example 3

**Preparation method:** In 10 ml of water, 14 mg of amikacin sulfate was dissolved to obtain a solution 1. In 30 ml of water, 427 mg of ethoxylated hydrogenated castor oil was dissolved to obtain a solution 2. The solution 1 and the solution 2 were mixed at a rate of 6 ml/min using a microfluidic device. The mixed solution was sterilized and filtered to obtain amikacin sulfate polymer-coated nanoparticles.

Stability investigation: Same as Example 4.

**Detection method:** Same as Example 4. The measured total drug concentration was 0.35388 mg/ml (0 days) and 0.34329 mg/ml (3 days), and the measured free drug concentration was 0.30611 mg/ml (0 days) and 0.32767 mg/ml (3 days). The results are shown in Table 3 below.

### Comparative Example 4

**Preparation method:** In 20 ml of water, 10 mg of oxaliplatin was dissolved to obtain a solution 1. In 10 ml of anhydrous ethanol, 120 mg of DSPC, 40 mg of cholesterol and 40 mg of mPEG2000-DSPE were dissolved to obtain a solution 2. The solution 1 and the solution 2 were mixed at a rate of 9 ml/min using a microfluidic device, and the mixed solution was diluted to 50 times the volume with a 1% sucrose solution and dialyzed twice using a 30 kD regenerated cellulose dialysis membrane to remove ethanol and perform buffer exchange. The first dialysis was performed at room temperature for 3 h, and the last dialysis was performed at 4°C overnight. The mixed solution was sterilized and filtered to obtain oxaliplatin liposomes.

**Detection method:** Same as Example 3. The measured total drug concentration was 0.34389 mg/ml, and the measured free drug concentration was 0.31005 mg/ml. The results are shown in Table 3 below.

### Comparative Example 5

**Preparation method:** In 10 ml of water, 38 mg of octreotide acetate and 18 mg of sodium alginate were dissolved to obtain a solution 1. In 10 ml of water, 14 mg of anionic surfactant sodium dodecyl sulfate was dissolved to obtain a solution 2. The solution 1 and the solution 2 were mixed at a rate of 9 ml/min using a microfluidic device. The mixed solution was sterilized and filtered to obtain octreotide nanoparticles.

Stability investigation: Same as Example 4.

Detection method: Same as Example 5. The measured total drug concentration was 1.9735 mg/ml (0 days) and 1.9411 mg/ml (3 days), and the measured free drug concentration was 0.028523 mg/ml (0 days) and 0.88104 mg/ml (3 days). The results are shown in Table 3 below.

### Comparative Example 6

**Preparation method:** As a solution 1, 10 ml of a 0.3 mg/ml siRNA solution was used. In 10 ml of water, 32 mg of DMG-PEG2000 was dissolved to obtain a solution 2. The solution 1 and the solution 2 were mixed at a rate of 12 ml/min using a microfluidic device. The mixed solution was sterilized and filtered to obtain siRNA polymer-coated nanoparticles.

**Detection method:** Same as Example 8. The measured total drug concentration was 0.15010 mg/ml, and the measured free drug concentration was 0.14412 mg/ml. The results are shown in Table 3 below.

### Comparative Example 7

**Preparation method:** As a solution 1, a siRNA solution was diluted to 0.1 mg/ml with a 50 mM pH 4 sodium citrate buffer. DOTAP, DOPE (dioleoyl phosphatidylethanolamine), cholesterol and DMG-PEG2000 were dissolved in anhydrous ethanol at a ratio of 50: 10: 38.5: 1.5 to obtain a 50 mM lipid ethanol solution, which was used as a solution 2. The solution 1 and the solution 2 were mixed at a rate of 18 ml/min using a microfluidic device, with a volume ratio of solution 1 to solution 2 of 3: 1. The mixed solution was diluted to 200 times the volume with a pH 7.4 PBS buffer and dialyzed twice using a 10 kD regenerated cellulose dialysis membrane to remove ethanol and perform buffer exchange. The first dialysis was performed at room temperature for 3 h, and the last dialysis was performed at 4°C overnight. The mixed solution was sterilized and filtered through a filter membrane to obtain siRNA lipid nanoparticles.

**Detection method:** Same as Example 8. The measured total drug concentration was 0.077142 mg/ml, and the measured free drug concentration was 0.0020593 mg/ml. The results are shown in Table 3 below.

### 1. Performance indicators

Performance parameters (average particle size, polydispersity index PDI, Zeta potential, and encapsulation efficiency) of the polymer-coated nanoparticles were tested according to the methods described in the examples and comparative examples. The results are shown in Table 3 below.

**Table 3 Performance data of polymer-coated nanoparticles**

| Test group | Average particle size nm | PdI | Zeta potential mV | Encapsulation efficiency % |
|---|---|---|---|---|
| Example 1 | 34.46 ± 0.33 | 0.41 ± 0.01 | -8.11 ± 0.90 | 99.6 |
| Example 2 | 19.26 ± 0.46 | 0.30 ± 0.01 | -13.7 ± 2.27 | 97.3 |
| Example 3 | 19.43 ± 0.36 | 0.16 ± 0.01 | -6.12 ± 0.54 | 87.3 |
| Example 4 | 12.74 ± 0.62 | 0.42 ± 0.02 | -20.4 ± 8.26 | 99.4 |
| Example 5 | 20.43 ± 0.64 | 0.26 ± 0.01 | -20.5 ± 1.84 | 97.9 |
| Example 6 | 25.30 ± 1.96 | 0.32 ± 0.03 | -8.72 ± 0.95 | 98.5 |
| Example 7 | 30.41 ± 0.48 | 0.22 ± 0.01 | -10.8 ± 0.79 | 99.8 |
| Example 8 | 390.5 ±5.78 | 0.28 ± 0.03 | 28.7 ± 0.64 | 75.8 |
| Example 9 | 24.72 ± 1.43 | 0.12 ± 0.05 | -2.98 ± 0.773 | 76.3 |
| Example 10 | 13.87 ± 0.37 | 0.16 ± 0.01 | -0.54 ± 0.82 | 96.4 |
| Example 11 | 23.85 ± 0.28 | 0.23 ± 0.01 | -6.71 ± 1.86 | 98.9 |
| Example 12 | 150.6 ± 0.25 | 0.20 ± 0.02 | 18.5 ± 0.43 | 85.7 |
| Example 13 | 290.5 ± 1.21 | 0.15 ± 0.02 | 25.5 ± 0.23 | 89.2 |
| Comparative Example 1 | 708.6 ± 80.42 | 0.73 ± 0.16 | -30.6 ±0.11 | 40.2 |
| Comparative Example 2 | 456.7 ± 3.87 | 0.68 ± 0.02 | -60.5 ± 0.65 | 24.9 |
| Comparative Example 3 | 16.94 ± 0.15 | 0.15 ± 0.01 | -6.67 ± 1.22 | 13.5 |
| Comparative Example 4 | 116.2 ± 0.75 | 0.32 ± 0.04 | -42.5 ± 6.04 | 9.8 |
| Comparative Example 5 | 195.3 ± 2.75 | 0.49 ± 0.51 | -56.4 ± 0.55 | 98.6 |
| Comparative Example 6 | 12.90 ± 0.22 | 0.05 ± 0.03 | -6.61 ± 1.36 | 4.0 |
| Comparative Example 7 | 104.4 ± 0.53 | 0.13 ± 0.05 | -3.55 ± 2.12 | 97.3 |

Stability investigation: Physiological conditions (pH 7.4, 10 mM phosphate + 150 mM NaCl) were simulated, and freeze-dried powder of the polymer-coated nanoparticles of the examples was reconstituted with a solution under the simulated physiological conditions and placed at 37°C for 3 days to investigate the in vitro stability of the polymer-coated nanoparticles.

**Table 4 Performance stability data of polymer-coated nanoparticles**

| Time | Time | Average particle size nm | PdI | Zeta potential mV | Encapsulation efficiency % |
|---|---|---|---|---|---|
| Example 4 | 0 hours | 12.74 ± 0.62 | 0.42 ± 0.02 | -20.4 ± 8.26 | 99.4 |
| | 3 days | 12.91 ± 0.52 | 0.38 ± 0.02 | -18.9 ± 1.45 | 98.6 |
| Example 5 | 0 hours | 20.43 ± 0.64 | 0.26 ± 0.01 | -20.5 ± 1.84 | 97.9 |
| | 3 days | 21.62 ± 0.45 | 0.28 ± 0.01 | -16.2 ± 1.14 | 96.5 |
| Example 8 | 0 hours | 390.5 ± 5.78 | 0.28 ± 0.03 | 28.7 ± 0.64 | 75.8 |
| | 3 days | 376.5 ± 4.32 | 0.19 ± 0.02 | 20.5 ± 0.46 | 79.8 |
| Example 10 | 0 hours | 13.87 ± 0.37 | 0.16 ± 0.01 | -0.54 ± 0.82 | 96.4 |
| | 3 days | 13.62 ± 0.09 | 0.11 ± 0.05 | -0.22 ± 0.45 | 96.2 |
| Example 12 | 0 hours | 150.6 ± 0.25 | 0.20 ± 0.02 | 18.5 ± 0.43 | 85.7 |
| | 3 days | 143.5 ± 0.18 | 0.15 ± 0.01 | 14.3 ± 0.38 | 88.4 |
| Comparative Example 3 | 0 hours | 16.94 ± 0.15 | 0.15 ± 0.01 | -6.67 ± 1.22 | 13.5 |
| | 3 days | 15.21 ± 0.23 | 0.17 ± 0.01 | -0.23 ± 0.25 | 4.5 |
| Comparative Example 5 | 0 hours | 195.3 ± 2.75 | 0.49 ± 0.51 | -56.4 ± 0.55 | 98.6 |
| | 3 days | 427.1 ± 6.23 | 0.63 ± 0.01 | -69.3 ± 1.15 | 54.6 |

### 2. Effect examples

### (1) Antibacterial activity test of polymer-coated nanoparticles of Examples 1 and 2 and Comparative Examples 1 and 2

A total of 44 strains of clinically isolated Gram-negative bacteria were collected for antimicrobial susceptibility testing, including 12 strains of Escherichia coli (carbapenem-susceptible), 12 strains of Klebsiella pneumoniae (carbapenem-susceptible), 10 strains of Acinetobacter baumannii (carbapenem-susceptible), and 10 strains of Pseudomonas aeruginosa (carbapenem-susceptible).

According to the recommendations of the 2021 edition of the Clinical and Laboratory Standards Institute (CLSI), a broth microdilution method was used to measure the minimum inhibitory concentration (MIC) of a test substance against clinical isolates.
(1) Preparation of antimicrobial drug: The test drug was dissolved and diluted, and the control drug was dissolved and diluted according to CLSI requirements. The test concentration range of the antibacterial drug solution was 0.015 mg/L to 32 mg/L.
(2) Culture medium: Cation adjusted muellerhinton broth (CAMHB), a product of BD Company, USA, batch number: 9324795.
(3) Inoculation amount: Fresh pure colonies on Columbia blood agar were picked using a direct colony suspension method. The colonies were adjusted to a McFarland turbidity of 0.5 and then appropriately diluted. The final concentration of the test bacteria was 10⁵ CFU/mL.
(4) Culture conditions: The colonies were cultured in air at 35°C ± 2°C for 16 to 20 hours.
(5) Reading and judgment of results: For colistin, according to the European EUCAST breakpoint judgment standard, a result of ≤ 2 mg/L is susceptible, and a result of ≥ 4 mg/L is resistant. The lowest antimicrobial drug concentration that inhibits bacterial growth is read as an MIC value.
(6) Quality control strains: Escherichia coli ATCC 25922 and Pseudomonas aeruginosa ATCC 27853.
(7) Data statistics: WHONET version 5.6 software was used to perform statistical analysis on the results of the antimicrobial susceptibility testing.

**Table 5 In vitro antimicrobial susceptibility testing of test drugs against 12 strains of Escherichia coli (MIC mg/L)**

| Bacteria (number of strains) | Test drug | MIC50 | MIC90 | Mode | Resistance rate (%) | Susceptibility rate (%) |
|---|---|---|---|---|---|---|
| Carbapenem-susceptible Escherichia coli (12) | Example 1 | 0.06 | 0.06 | 0.06 | 0 | 100 |
| | Example 2 | 0.03 | 0.06 | 0.03 | 0 | 100 |
| | Comparative Example 1 | 0.125 | 0.25 | 0.125 | 0 | 100 |
| | Comparative Example 2 | 0.125 | 0.25 | 0.125 | 0 | 100 |
| | Colistin | 0.125 | 0.25 | 0.125 | 0 | 100 |
| | sulfate | | | | | |

**Table 6 In vitro antimicrobial susceptibility testing of test drugs against 12 strains of Klebsiella pneumoniae (MIC mg/L)**

| Bacteria (number of strains) | Test drug | MIC50 | MIC90 | Mode | Resistance rate (%) | Susceptibility rate (%) |
|---|---|---|---|---|---|---|
| Carbapenem-susceptible Klebsiella pneumoniae (12) | Example 1 | 0.125 | 0.125 | 0.125 | 0 | 100 |
| | Example 2 | 0.06 | 0.06 | 0.06 | 0 | 100 |
| | Comparative Example 1 | 0.25 | 0.25 | 0.25 | 0 | 100 |
| | Comparative Example 2 | 0.5 | 0.5 | 0.5 | 0 | 100 |
| | Colistin sulfate | 0.5 | 0.5 | 0.5 | 0 | 100 |

**Table 7 In vitro antimicrobial susceptibility testing of test drugs against 10 strains of Acinetobacter baumannii (MIC mg/L)**

| Bacteria (number of strains) | Test drug | MIC50 | MIC90 | Mode | Resistance rate (%) | Susceptibility rate (%) |
|---|---|---|---|---|---|---|
| Carbapenem-susceptible Acinetobacter baumannii (10) | Example 1 | 0.125 | 0.25 | 0.125 | 0 | 100 |
| | Example 2 | 0.125 | 0.125 | 0.125 | 0 | 100 |
| | Comparative Example 1 | 0.5 | 0.5 | 0.5 | 0 | 100 |
| | Comparative Example 2 | 1 | 1 | 1 | 0 | 100 |
| | Colistin sulfate | 0.5 | 0.5 | 0.5 | 0 | 100 |

**Table 8 In vitro antimicrobial susceptibility testing of test drugs against 10 strains of Pseudomonas aeruginosa (MIC mg/L)**

| Bacteria (number of strains) | Test drug | MIC50 | MIC90 | Mode | Resistance rate (%) | Susceptibility rate (%) |
|---|---|---|---|---|---|---|
| Carbapenem-susceptible Pseudomonas aeruginosa (10) | Example 1 | 0.5 | 1 | 0.5 | 0 | 100 |
| | Example 2 | 0.25 | 1 | 0.5 | 0 | 100 |
| | Comparative Example 1 | 1 | 2 | 2 | 0 | 100 |
| | Comparative Example 2 | 2 | 2 | 2 | 0 | 100 |
| | Colistin sulfate | 1 | 2 | 2 | 0 | 100 |

**Table 9 In vitro antimicrobial susceptibility testing of test drugs against 44 strains of Gram-negative bacteria (MIC mg/L)**

| Bacteria (number of strains) | Test drug | MIC50 | MIC90 | Mode | Resistance rate (%) | Susceptibility rate (%) |
|---|---|---|---|---|---|---|
| Carbapenem-susceptible Gram-negative bacteria (44) | Example 1 | 0.125 | 0.5 | 0.06 | 0 | 100 |
| | Example 2 | 0.06 | 0.5 | 0.06 | 0 | 100 |
| | Comparative Example 1 | 0.5 | 2 | 0.5 | 0 | 100 |
| | Comparative Example 2 | 1 | 2 | 1 | 0 | 100 |
| | Colistin sulfate | 0.5 | 2 | 0.5 | 0 | 100 |

As shown in the above results, the antibacterial activity of Examples 1 and 2 against Gram-negative bacteria is better than that of colistin sulfate, while the antibacterial activity of Comparative Examples 1 and 2 against Gram-negative bacteria is equivalent to or weaker than that of colistin sulfate, showing that the polymer-coated nanoparticles of the present invention can significantly improve the antibacterial activity of drugs. The antibacterial activity of Comparative Example 1 against Gram-negative bacteria is not significantly different from that of colistin sulfate, and the antibacterial activity of Comparative Example 2 against Gram-negative bacteria is slightly weaker than that of colistin sulfate.

### (2) Safety test of polymer-coated nanoparticles of Examples 1 and 2 and Comparative Examples 1 and 2

The drug safety of colistin sulfate, the nanoparticles of Examples 1 and 2, and the nanoparticles of Comparative Examples 1 and 2 in mice was compared. Male ICR mice (purchased from Shanghai Lingchang Experimental Animal Co., Ltd.), 7 to 8 weeks old and weighing 25 g to 30 g, were given colistin sulfate or a polymyxin polymer-coated nanoparticle solution by intravenous injection at an administration concentration of 1.5 mg/ml and a dose range of 8 mg/kg to 60 mg/kg. The dose volume was adjusted according to the weight of the animal. The highest dose that enables all subjects in the test to survive continuously is defined as MTD.

Animal grouping: Before starting the administration, the animals were weighed and randomly grouped according to the weight.

Test indicators: clinical observation, animal morbidity/death, and weight change. The maximum compound dosage that causes the weight loss of mice to not exceed 20% and no animal death is the maximum tolerated dose.

The results are shown in the table below.

**Table 10 Safety test results**

| Investigation items | Colistin sulfate | Example 1 | Example 2 | Comparative Example 1 | Comparative Example 2 |
|---|---|---|---|---|---|
| MTD/mg/kg | 8 | ≥ 60 | ≥ 50 | 12 | 8 |

Based on the above results, it can be seen that the maximum tolerated dose of the polymer-coated nanoparticles of Examples 1 and 2 in mice is much higher than that of colistin sulfate, while the maximum tolerated dose of Comparative Examples 1 and 2 is equivalent to that of colistin sulfate. The above results show that the polymer-coated nanoparticles of the present invention can significantly improve the safety of drugs in mice, and the safety of drugs with narrow therapeutic windows is greatly improved.

### (3) The effect of Example 3 on the proliferation of HT29 cells in vitro was investigated, and the IC50 activity value of the test substance was tested.

Cell culture: HT29 (Cat. No.: CBP30001L, purchased from Cobioer) was revived and cultured with 1640+10% FBS.

Plating: Normally growing cells were digested with a trypsin cell digestion solution, then centrifuged, counted, and plated into a 96-well plate at a cell density of 3000 cells/well, 100 µL per well, for a total of 60 wells, and sealed with an appropriate amount of PBS around to prevent water evaporation in the edge wells.

Administration: The administration was performed on the second day after cell plating, 100 µL of a drug solution was added to each well, and two replicate wells were set up for each concentration point.

Detection: After 72 hours of drug treatment, all the liquid in the 96-well plate was poured out, and 100 µL of a CCK8 (diluted to 1: 9 with culture medium) solution was added to each well. After incubation at 37°C for a period of time (OD450 of Vehicle group reached above 1.0), the absorption at 450 nm was detected using an ELISA reader.

Data processing: Inhibition Rate (%) = (1 - OD450_{Sample}/OD450_{Vehicle}) × 100, where OD450_{Sample} is the 450nm absorption value of the drug-treated group, and OD450_{Vehicle} is the 450nm absorption value of the DMSO control group. GraphPad Prism 8.0 software was used to draw an S-shaped dose-survival rate curve with a nonlinear regression model, and the IC50 value was calculated.

### Test results:

**Table 11 IC50 activity values**

| Investigation items | Cyclohexanediamine platinum dihydrate | Example 3 |
|---|---|---|
| HT-29ReIC50 µM | 1.61 ± 0.23 | 0.60 ± 0.19 |

The above results show that the polymer-coated nanoparticles of the present invention have a stronger inhibitory effect on the proliferation of HT-29 cells in vitro than platinum cyclohexanediamine dihydrate.

### (4) Investigation of safety test of polymer-coated nanoparticles of Example 3

Male Balb/c nude mice (purchased from Shanghai Lingchang Experimental Animal Co., Ltd.), 6 to 7 weeks old and weighing 17 g to 21 g, were given platinum cyclohexanediamine dihydrate or a platinum cyclohexanediamine dihydrate polymer-coated nanoparticle solution by intravenous injection at an administration concentration of 1.0 mg/ml. The dose volume was adjusted according to the weight of the animal. The highest dose that enables all subjects in the test to survive continuously is defined as MTD.

Animal grouping: Before starting the administration, the animals were weighed and randomly grouped according to the weight.

Test indicators: After administration, the mice were observed continuously for at least 14 days, and the observation interval and frequency were properly arranged so as to observe the occurrence of toxic reactions, recovery time, animal death time, etc. If the toxic reactions of the mice occur slowly or the mice recover slowly, the observation time should be appropriately extended.

The results are shown in the table below.

**Table 12 Test results**

| Investigation items | Cyclohexanediam ine platinum dihydrate | Example 3 |
|---|---|---|
| MTD/mpk | <4 | 8 |

The results show that the tolerance of mice to the polymer-coated nanoparticles of Example 3 is much higher than that to platinum cyclohexanediamine dihydrate, showing that the polymer-coated nanoparticles of the present invention greatly improve the safety of platinum cyclohexanediamine dihydrate.

### (5) Investigation of in vitro release of Examples 4, 5, 6, 7, and 11

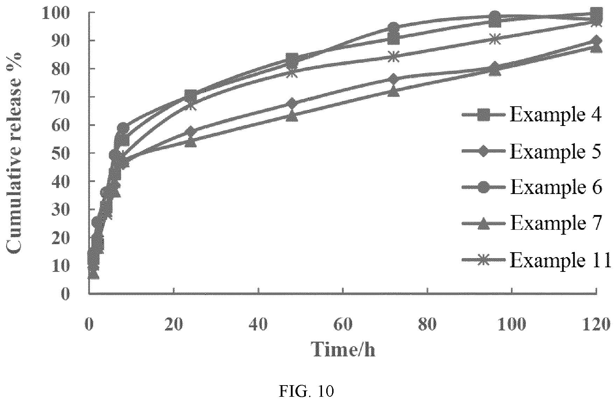

In dialysis bags (molecular weight cut-off: 100 kD), 1.5 ml of each polymer-coated nanoparticle solution was placed. Each of the dialysis bags was placed in a centrifuge tube containing 18.5 ml of PBS (pH 7.4, 10 mM phosphate + 150 mM NaCl) and shaken in a 37°C constant temperature water bath oscillator (100 rpm). A sample was taken at regular intervals and the dialysis bag was transferred to a fresh medium. The concentration of the free drug outside the dialysis bag was measured by an HPLC method, and the release of the polymer-coated nanoparticles at each time point was calculated. The release curve was shown in FIG. 10.

The polymer-coated nanoparticles provided by the present invention can be released stably and continuously in a PBS (pH 7.4, 10 mM phosphate + 150 mM NaCl) buffer.

The above detailed description is a specific description of one feasible embodiment of the present invention. The embodiment is not intended to limit the patent scope of the present invention. Any equivalent implementation or modification that does not deviate from the present invention should be included in the scope of the technical solution of the present invention.

## Claims

1. Polymer-coated nanoparticles having a structure comprising: a hydrophilic coacervate core, and a coating of a polymer molecular material containing a polyoxyethylene structural unit, wherein
the hydrophilic coacervate core includes a cationic component and an anionic component as preparation raw materials,
the cationic component is a cation, a compound that is ionizable into a cation, or a combination thereof,
the anionic component is an anion, a compound that is ionizable into an anion, or a combination thereof, and
the cationic compound or the combination thereof is any one or more selected from hydrophilic small-molecule compounds or polypeptides that are ionizable or net positively charged.

2. The polymer-coated nanoparticles according to claim 1, wherein
the nanoparticles have a particle size of 10 nm to 400 nm, a PDI of less than 0.5, a weakly charged or electrically neutral surface, a surface potential of -40 mV to 40 mV, and an encapsulation efficiency greater than 60%.

3. The polymer-coated nanoparticles according to claim 1, wherein
the polypeptides are ionizable or net positively charged hydrophilic polypeptides with a molecular weight less than 10,000, and the ionizable or net positively charged hydrophilic small-molecule compounds are ionizable or net positively charged hydrophilic compounds containing an amine structure.

4. The polymer-coated nanoparticles according to claim 3, wherein the polypeptides include any one or more of:
an ionizable hydrophilic polypeptide having a cyclic structure containing a free amine group, and derivatives thereof,
an ionizable hydrophilic polypeptide having a molecular weight less than 10,000 and having a free primary amine, secondary amine, or tertiary amine structure, and derivatives thereof,
polylysine and derivatives thereof,
polyarginine and derivatives thereof,
polyhistidine and derivatives thereof, and
an arginine-rich polypeptide.

5. The polymer-coated nanoparticles according to claim 4, wherein
the polypeptide is selected from the group consisting of polymyxin and derivatives thereof, daptomycin, vancomycin, tigecycline, thymalfasin, octreotide acetate, liraglutide, and pasireotide.

6. The polymer-coated nanoparticles according to claim 1, wherein
the ionizable or net positively charged hydrophilic small-molecule compounds include any one or more of: a metal complex and derivatives thereof, an ionizable hydrophilic anesthetic/analgesic drug containing an amine structure, an ionizable hydrophilic psychotropic drug containing an amine structure, an ionizable hydrophilic anti-cancer or anti-tumor drug containing an amine structure and derivatives thereof, imipenem, clindamycin hydrochloride, and amikacin sulfate.

7. The polymer-coated nanoparticles according to claim 6, wherein
the metal complex and the derivative thereof are selected from the group consisting of a platinum complex and derivatives thereof, a ruthenium (II) complex and derivatives thereof, and a vanadium complex and derivatives thereof,
the ionizable hydrophilic anesthetic/analgesic drug containing an amine structure is selected from the group consisting of clonidine hydrochloride and pregabalin,
the ionizable hydrophilic psychotropic drug containing an amine structure is selected from the group consisting of risperidone hydrochloride, methylphenidate, levetiracetam, and brivaracetam, and
the ionizable hydrophilic anti-cancer or anti-tumor drug containing an amine structure and the derivative thereof are selected from the group consisting of bortezomib, imatinib, and palbociclib.

8. The polymer-coated nanoparticles according to claim 1, wherein
the anionic compound or the combination thereof is any one or more of an ionizable or anionic hydrophilic small-molecule compound, an ionizable or anionic hydrophilic monosaccharide or oligosaccharide acid and derivatives thereof, an ionizable or anionic hydrophilic polysaccharide and derivatives thereof, an anionic polyamino acid and derivatives thereof, and a nucleic acid.

9. The polymer-coated nanoparticles according to claim 8, wherein
the monosaccharide or oligosaccharide acid and the derivatives thereof include any one or more of sulfated glucose, sulfated fructose, phosphorylated glucose, phosphorylated fructose, sulfated sucrose, sulfated lactose, sulfated trehalose, phosphorylated sucrose, phosphorylated lactose, and phosphorylated trehalose.

10. The polymer-coated nanoparticles according to claim 9, wherein
the polysaccharide and the derivatives thereof are any one or more of glycosaminoglycan, cellulose polysaccharide, polyuronic acid polysaccharide, and galactomannan polysaccharide and derivatives thereof.

11. The polymer-coated nanoparticles according to claim 10, wherein
the glycosaminoglycan is any one or more of sodium hyaluronate, cross-linked sodium hyaluronate, chondroitin sulfate, dermatan sulfate, keratan sulfate, heparin sodium, heparan sulfate, dalteparin sodium, enoxaparin sodium, and fondaparinux sodium.

12. The polymer-coated nanoparticles according to claim 10, wherein
the cellulose polysaccharide is any one or more of cellulose acetate, sodium carboxymethyl cellulose, hydroxyethyl cellulose, hydroxyethyl methyl cellulose, hydroxypropyl cellulose, and hydroxypropyl methyl cellulose.

13. The polymer-coated nanoparticles according to claim 10, wherein
the polyuronic acid polysaccharide and galactomannan polysaccharide and the derivatives thereof are any one or more of polyglucuronic acid, polyuronide, sodium polymannosuronate, sodium polyguluronate, alginic acid, sodium alginate, propylene glycol alginate, xanthan gum, tragacanth gum, arabic gum, and carrageenan.

14. The polymer-coated nanoparticles according to claim 8, wherein
the nucleic acid is ribonucleic acid or deoxyribonucleic acid, including any one or more of siRNA, mRNA, miRNA, antisense oligonucleotide, DNA, circular RNA, and tRNA.

15. The polymer-coated nanoparticles according to any one of claims 1 to 14, wherein
the anionic component includes any one or more of polyglutamic acid polypeptide and derivatives thereof, polyaspartic acid polypeptide and derivatives thereof, hyaluronate, sucrose octasulfate, sodium alginate, xanthan gum, CMC-Na, siRNA, mRNA, sulfadiazine sodium, and sodium carboxymethyl cellulose.

16. The polymer-coated nanoparticles according to claim 1, wherein
the polyoxyethylene structural unit contained is -(CH₂CH₂O)n-, wherein n = 6 to 460.

17. The polymer-coated nanoparticles according to claim 1, wherein
the polymer molecular material containing a polyoxyethylene structural unit is any one or more of PEG-phospholipid, PEG-cholesterol, PEG-polymer, polysorbate, polyoxyethylene castor oil and derivatives thereof, and polyoxyethylene stearate.

18. The polymer-coated nanoparticles according to claim 17, wherein
the polymer molecular material containing a polyoxyethylene structural unit includes any one or more of 1,2-distearoyl-*rac*-glycerol-3-phosphatidylethanolamine-polyethylene glycol, PEG-PLA, 1,2-dimyristoyl-*rac*-glycerol-3-methoxypolyethylene glycol, polyethylene glycol-polylactic acid-glycolic acid polymer, polyglutamic acid-polyethylene glycol-carboxylic acid, polysorbate, polyoxyethylene castor oil, polyoxyethylene stearate, polyethylene glycol-polyglutamic acid, polyethylene glycol-dipalmitin, polyethylene glycol-dipalmitoyl phosphatidylethanolamine, polyethylene glycol-dimyristoyl phosphatidylethanolamine, polyethylene glycol-dilauroyl phosphatidylethanolamine, polyethylene glycol-dipalmitoyl phosphatidylserine, polyethylene glycol-cholesterol, and cholesterol-polyethylene glycol-VA.

19. The polymer-coated nanoparticles according to claim 1, wherein
in the nanoparticles, the charge ratio of the cationic component to the anionic component is (1 to 8) : (1 to 10), and the content of the hydrophilic coacervate core in the nanoparticle is 1% to 90% by mass, preferably from 5% to 80% by mass.

20. A method for preparing the polymer-coated nanoparticles according to any one of claims 1 to 19, the method comprising:
(1) dissolving and mixing the cationic component and the anionic component to obtain a coacervate solution;
(2) dissolving the polymer molecular material containing a polyoxyethylene structural unit, to obtain a coating solution; and
(3) mixing the coacervate solution and the coating solution to obtain a polymer-coated nanoparticle solution.

21. The method according to claim 20, further comprising
filtering and/or purifying the polymer-coated nanoparticle solution.

22. The method according to claim 20, wherein
during the preparation of the nanoparticles, the coacervate solution is a homogeneous solution, and the mixing ratio of the coacervate solution to the coating solution is (1 to 10) : (1 to 20) by volume.

23. The method according to claim 20, wherein
the mixing is performed by a microchannel mixing method, and the microchannel mixing method includes an injection technique, a microfluidic technique, or a jet flow technique.

24. The method according to claim 23, wherein
the microchannel mixing has a mixing speed of is 0.5 ml/min to 400 ml/min.

25. A preparation of the polymer-coated nanoparticles according to any one of claims 1 to 19 or a preparation of nanoparticles prepared by the method according to any one of claims 20 to 24, wherein
the nanoparticles have a structure comprising: a hydrophilic coacervate core containing an active ingredient, and a coating of a polymer molecular material containing a polyoxyethylene structural unit,
the nanoparticles have an encapsulation efficiency greater than 60%, an average particle size of 10 nm to 400 nm, a weakly charged or electrically neutral surface, and a surface potential of -40 mV to 40 mV

26. The preparation of nanoparticles according to claim 25, further comprising an excipient commonly used in pharmaceutics including any one or more of potassium dihydrogen phosphate, disodium hydrogen phosphate, sodium dihydrogen phosphate, dipotassium hydrogen phosphate, sodium chloride, potassium chloride, sodium acetate, acetic acid, sucrose, mannitol, lactose, glucose, trehalose, polyethylene glycol, glycerol, a phosphate salt, an acetate salt, amino acids, water for injection, a 0.5% to 0.9% sodium chloride solution, a 1% to 5% glucose solution, and a PBS buffer.
